# EUROPÄISCHE PATENTANMELDUNG

(11) **EP 0 679 625 A2**
(43) Veröffentlichungstag der Anmeldung: **02.11.1995**
(21) Anmeldenummer: 95105849.4
(22) Anmeldetag: 19.04.1995
(51) Int. Cl.: C07C 37/06, C07C 39/07

(54) **Verfahren zur Herstellung von 2,3,6-Trimethyl-phenol**

(30) Priorität: 28.04.1994 DE 4414877
(71) Anmelder: BASF AKTIENGESELLSCHAFT, D-67056 Ludwigshafen (DE)
(72) Erfinder: Rittinger, Stefan, Dr., D-68199 Mannheim (DE); Rieber, Norbert, Dr., D-68259 Mannheim (DE); Arnold, Lothar, Dr., D-69118 Heidelberg (DE); Hörcher, Ulrich, Dr., D-68199 Mannheim (DE)

(57) **Zusammenfassung**

Verfahren zur Herstellung von 2,3,6-Trimethylphenol das dadurch gekennzeichnet ist, daß man Diethylketon mit einem 1-Amino-vinyl-methylketon mit Vorteil in Gegenwart von Basen bei Temperaturen von 50 bis 200°C umsetzt.

## Beschreibung

Die vorliegende Erfindung betrifft ein Verfahren zur Herstellung von 2,3,6-Trimethyl-phenol, einem essentiellen Vorprodukt für die Herstellung von Vitamin E.

2,3,6-Trimethyl-phenol kann gemäß DE 2 415 930 durch katalytische Alkylierung von n-Kresol in der Gasphase erhalten werden. Nachteilig an diesem Verfahren ist, daß hierbei Isomerengemische anfallen, aus denen das gewünschte Isomere nur schwierig und unter Verlusten in reiner Form gewonnen werden kann.

Weitere technisch ausgeübte Verfahren beruhen auf der Kondensation von Diethylketon mit einem C₄-Baustein, wie Crotonaldehyd (vgl. DE 1 793 037) oder Methylvinylketon (vgl. DE 1 668 874). Nachteilig an diesen an sich vorteilhaften Verfahren ist, daß man hierbei nicht direkt zu dem 2,3,6-Trimethyl-phenol gelangt, sondern zunächst zu dem Trimethylcyclohexenon, welches in einer zusätzlichen Stufe zum Trimethylphenol dehydriert werden muß.

Es war daher die Aufgabe der Erfindung ein Verfahren zur Herstellung von 2,3,6-Trimethyl-phenol ausgehend von Diethylketon und einem leicht zugänglichen C₄-Baustein zu entwickeln, das mit einer einzigen Reaktionsstufe auskommt.

Gegenstand der Erfindung ist ein Verfahren zur Herstellung von 2,3,6-Trimethylphenol das dadurch gekennzeichnet ist, daß man Diethylketon mit einem 1-Amino-vinylmethylketon bei Temperaturen von 50 bis 200°C, vorzugsweise 70 bis 150°C, umsetzt.

Die Umsetzung erfolgt also nach folgendem Reaktionsschema: 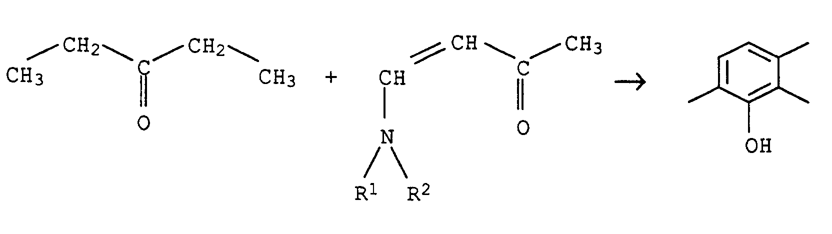
Als 1-Amino-vinylmethylketone können prinzipiell alle 1-Aminovinylmethylketone verwendet werden, die eine primäre, sekundäre oder tertiäre Aminogruppe aufweisen. Aus wirtschaftlichen Gründen wird man bevorzugt solche Aminoverbindungen verwenden, die kostengünstig zugängig und/oder besonders einfach zu handhaben oder abzutrennen sind. Genannt seien beispielsweise 1-Dimethylamino-, 1-Diethylamino-, 1-Dipropylamino-, 1-Dicyclohexylamino-, 1-Diethanolamino-, 1-Piperazino-, 1-N-Methyl-piperazino- oder 1-Morpholinamino-vinylmethylketon. Prinzipiell können jedoch alle 1-Amino-vinylmethylketone der oben angegebenen Formel eingesetzt werden, in denen die Substituenten R¹ und R² folgende Bedeutung haben:
a) R¹ und R² unabhängig voneinander Wasserstoff oder
   C₁- bis C₂₀-Alkyl, vorzugsweise C₁- bis C₈-Alkyl, insbesondere C₁- bis C₄-Alkyl wie Methyl, Ethyl, n-Propyl, iso-Propyl, n-Butyl, iso-Butyl und tert.-Butyl;
   C₃- bis C₈-Cycloalkyl, vorzugsweise C₅- bis C₈-Cycloalkyl wie Cyclopentyl, Cyclohexyl, Cycloheptyl und Cyclooctyl, insbesondere Cyclopentyl und Cyclohexyl;
   C₁- bis C₂₀-Hydroxyalkyl, vorzugsweise C₂- bis C₈-Hydroxyalkyl, insbesondere C₂- bis C₄-Hydroxyalkyl wie 2-Hydroxyethyl und 3-Hydroxy-propyl;
   Phenyl und C₇- bis C₁₂-Alkylphenyl wie 2-Methyl-phenyl, 3-Methyl-phenyl, 4-Methyl-phenyl, 2,4-Dimethyl-phenyl, 2,5-Dimethyl-phenyl, 2,6-Dimethyl-phenyl, 3,5-Dimethyl-phenyl und 2,3,4-Trimethyl-phenyl;
   oder
   C₇- bis C₁₂-Phenylalkyl wie Benzyl, Phenethyl, 1-Phenyl-propyl, 2-Phenyl-propyl, 1-Phenyl-butyl, 2-Phenyl-butyl, 3-Phenyl-butyl, vorzugsweise Benzyl- und Phenethyl;
   oder aber
b) R¹ und R² stehen gemeinsam für eine C₂- bis C₇-Alkylenkette, die durch ein bis vier C₁- bis C₄-Alkylreste substituiert und/oder durch Sauerstoff, Stickstoff oder Schwefel unterbrochenen sein kann. Genannt seien beispielsweise folgende Gruppen:
   -(CH₂)₂-, -(CH₂)₃-, -(CH₂)₄-, -(CH₂)₅-, -(CH₂)₆-, -(CH₂)₇-, -(CHCH₃)-, -(CH₂)₂-O-(CH₂)₂-, -(CH₂)₂-N-(CH₂)₂- und -(CH₂)₂-S-(CH₂)₂-.

Da kürzlich ein einfaches Verfahren zur Herstellung für die 1-Amino-vinylmethylketone durch Umsetzen von Diacetylene enthaltenden, bei der Acetylenerzeugung anfallenden Crackgasen, mit sekundären Aminen und Wasser bei Temperaturen von 0 bis 150°C gefunden wurde (vgl. DE-Patentanmeldung P 43 08 080.4) ergibt sich somit ein sehr vorteilhaftes Verfahren, bei dem aus einfach zugänglichen Ausgangsstoffen direkt das begehrte 2,3,6-Trimethyl-phenol erhalten werden kann.

Weiterhin kann man die 1-Amino-vinylmethylketone beispielsweise gemäß US 3,141,880 durch Umsetzen von Enaminen mit Keton oder gemäß US 3,285,915 durch Umsetzen von sekundären Aminen mit einem 3-Alkoxycyclobutanon herstellen.

Das erfindungsgemäße Verfahren wird mit Vorteil in Gegenwart basischer Mittel durchgeführt.

Als basische Mittel können alle stärkeren Basen, die deprotonierend wirken können, verwendet werden. Genannt seien beispielsweise Hydroxide, Oxide, Alkoholate, Amide oder Hydride von Alkali- oder Erdalkalimetallen, metallorganische Verbindungen, wie Butyllithium oder Phenyllithium oder quartäre Ammoniumhydroxide, wie Benzyltrimethylammoniumhydroxid verwendet werden.

Die basischen Mittel werden im allgemeinen in stöchiometrischen Mengen oder gar in einem geringen Überschuß eingesetzt.

Die erfindungsgemäße Umsetzung kann sowohl in Gegenwart als auch in Abwesenheit eines Lösungsmittels durchgeführt werden. Bei Abwesenheit eines Lösungsmittels arbeitet man vorteilhaft in überschüssigem Diethylketon als Verdünnungsmittel.

Als Lösungsmittel kommen beispielsweise in Betracht: aliphatische Petrolether, Kohlenwasserstoffe, wie Petrolether oder Benzingemische; cycloaliphatische Kohlenwasserstoffe, wie Cyclohexan; aromatische Kohlenwasserstoffe, wie Benzol, Toluol, Xylol, Cumol und p-Diisopropylbenzol; chlorierte Kohlenwasserstoffe, wie Chlorbenzol, Chlortoluol und Dichlorbenzol; Ether, wie Methyl-tert.-butyl-ether, Tetrahydrofuran und Dioxan; Sulfoxide, wie Dimethylsulfoxid, Säureamide, wie Dimethylformamid und N-Methylpyrrolidon, und höhere Alkohole, wie Pentanole, in Betracht.

Mit besonderem Vorteil gelingt die Umsetzung bei Verwendung von mit Wasser nicht mischbaren Lösungsmitteln.

Bei Verwendung dieser nicht mit Wasser mischbaren Lösungsmittel erweist es sich als vorteilhaft, unter Auskreisen von Wasser zu arbeiten.

Besonders vorteilhaft an dem erfindungsgemäßen Verfahren ist, daß auch Gemische sekundärer Amine mit Wasser eingesetzt werden können, da hierdurch die Aminovinylketone direkt, d.h. ohne Isolierung, nach ihrer Herstellung aus Diacetylenen und der wäßrigen Lösung eines sekundären Amins gemäß P 43 08 080.4 zur Herstellung von Trimethylphenol eingesetzt werden können.

Die Kondensation wird im allgemeinen bei Temperaturen von 50 bis 200°C, vorzugsweise 70 bis 150°C ausgeführt.

Die Reaktionszeiten betragen im allgemeinen - je nach Reaktionstemperatur und Reaktionspartner - 20 bis 40 Stunden, vorzugsweise 25 bis 30 Stunden.

In der Regel wendet man Atmosphärendruck an. Man kann aber auch unter vermindertem oder unter erhöhtem Druck arbeiten.

Diethylketon und das Aminovinylmethylketon verwendet man zweckmäßig in einem Molverhältnis von 1:1 bis 50:1, vorzugsweise 1:1 zu 20:1.

Zur Aufarbeitung des Reaktionsgemisches geht man im allgemeinen so vor, daß man zunächst das entstandene Phenolat neutralisiert und anschließend das Reaktionsgemisch durch fraktionierte Destillation auftrennt.

Das durch Destillation oder aus dem Destillationssumpf erhaltene Amin kann wieder zur Herstellung des 1-Amino-vinylmethylketons eingesetzt werden.

Mit Hilfe des erfindungsgemäßen Verfahrens kann das als essentielles Vitamin-E-Vorprodukt begehrte 2,3,6-Trimethyl-phenol in einer einzigen einfachen Reaktionsstufe aus dem gut zugänglichen Diethylketon und aus den auf einfache Weise und gut zugänglichen Aminovinylmethylketonen der Formel III in recht guten Ausbeuten erhalten werden.

### Beispiel

In einem 250 ml Glaskolben mit Rührer und aufgesetztem Wasserauskreiser wurden 16,9 g (0,1 mol) Dipropylaminovinylmethylketon, 172,3 g (2 mol) Diethylketon und 11,2 g einer 50 %igen wäßrigen KOH-Lösung (entsprechend 0,1 mol) vermischt und 29 Stunden unter Rückfluß und Auskreisung von Wasser zum Sieden erhitzt. Dabei wurden 6,7 ml H₂O abgeschieden (erwartet: 5,6 ml aus der Kalilauge, 1,8 ml aus der Reaktion). Die erhaltene braunschwarze Reaktionslösung wurde nach Neutralisation mit Essigsäure gaschromatographisch analysiert. Der Gehalt an 2,3,6-Trimethyl-phenol wurde mittels Faktorenbildung quantifiziert. Die Selektivität zu Trimethylphenol betrug 63 % bezogen auf umgesetztes Aminovinylmethylketon (Umsatz 89 %). Wichtigstes Nebenprodukt war 3-Ethyl-4-methyl-phenol mit einer Selektivität von 26 % (nach GC-Flächen-%).

## Patentansprüche

1. Verfahren zur Herstellung von 2,3,6-Trimethylphenol, dadurch gekennzeichnet, daß man Diethylketon mit einem 1-Amino-vinylmethylketon bei Temperaturen von 50 bis 200°C umsetzt.

2. Verfahren gemäß Anspruch 1, dadurch gekennzeichnet, daß man Diethylketon mit dem 1-Amino-vinylmethylketon bei Temperaturen von 70 bis 150°C umsetzt.

3. Verfahren gemäß Anspruch 1, dadurch gekennzeichnet, daß man das Diethylketon in Gegenwart einer Base, die deprotonierend wirken kann, mit dem 1-Amino-vinylmethylketon umsetzt.

4. Verfahren gemäß Anspruch 3, dadurch gekennzeichnet, daß man als Base ein Hydroxid, Oxid, Alkoholat, Amid oder Hydrid eines Alkali- oder Erdalkalimetalles verwendet.

5. Verfahren gemäß Anspruch 1, dadurch gekennzeichnet, daß man als 1-Amino-vinyl-methylketon 1-Dimethylamino-, 1-Diethylamino-, 1-Morpholinamino-, 1-Dipropylamino-, 1-Dicyclohexylamino-, 1-Diethanolamino-, 1-Piperazino- oder 1-N-Methylpiperazino-vinylmethylketon verwendet.
